# EUROPEAN PATENT APPLICATION

(11) **EP 1 537 895 A1**
(43) Date of publication of application: **08.06.2005**
(21) Application number: 04106016.1
(22) Date of filing: 23.11.2004
(51) Int. Cl.: A61N 1/372, A61N 1/375, A61B 5/00

(54) **Implantable electromedical device with an antenna system**

(30) Priority: 03.12.2003 IT BO20030732
(71) Applicant: Tre Esse Progettazione Biomedica S.r.l, 40138 Bologna (IT)
(72) Inventor: Plicchi, Gianni, 40136, Bologna (IT); Marcelli, Emanuela, 62100, Macerata (IT)
(74) Representative: Porsia, Dino, Dr.

(57) **Abstract**

Implantable electro-medical devices which can be implanted in the human body, such as pacemakers, defibrillators or the like, comprising an antenna system so as to allow dialogue between the transceiver system inside these said devices and transceiver devices situated outside and also relatively distant from the patient's body. The implantable devices have a box-shaped body (C1) which is made for example of titanium and contains the various electronic components and which is closed by a cover (C10) provided with openings from which the terminals for the stimulation and-/or control electrode or electrodes project. This cover has situated above it and is sealed in a fluid-tight manner by a cap (C2) which is made of resin or silicone or derivatives thereof and through which said terminals pass in a fluid-tight manner. The system comprises at least one antenna of the cylindrical helix type (3) able to operate at frequencies of between 402 and 405 MHz and on the cover (C10) of the said container (C1) there is formed at least one window (1) of suitable size and shape for allowing communication, with the exterior, of the said antenna system which comprises an interface (2) inside the container (C1) and the said cylindrical-helix antenna (3) which may be housed completely inside the resinous cap (C2) or partly inside the resinous cap and partly inside the metallic container (C1) or completely inside this container. The said hole (1) for receiving the antenna or allowing through the electric field produced by the said antenna or directed towards it, is closed by an insert (4) which is made of any gas-proof and moisture-proof material permeable to the radiofrequencies in question and can be fixed in a fluid-tight manner on the said cover (C10).

## Description

The invention concerns an antenna system for electro-medical devices which can be implanted in the human body, such as pacemakers, defibrillators or the like, in order to allow dialogue between the transceiver system inside the said devices and transceiver devices situated outside and also relatively distant from the patient's body, for example for remote reception/transmission of data to and from monitoring stations or to or from control and/or programming stations. The antenna system in question must be able to operate within the range of the frequency assigned to the devices in question, which is currently between 402 and 405 MHz.

The implantable devices of the type in question usually have a box-shaped body which is made of titanium and contains the various electronic components and which has a cover with openings from which the terminals for the stimulation and/or control electrode or electrodes project, this cover having situated above it and being sealed in a fluid-tight manner by a cap which is made of resin or silicone or derivatives thereof and through which said terminals pass in a fluid-tight manner. The antenna systems for these devices may not be housed completely inside the said box-shaped body made of titanium, since this material screens the radiofrequency emission, for which reason the said antenna systems are all or partly housed inside the said resin or silicone cap. These synthetic materials, with the passing of time, inevitably become saturated with moisture and modify their electric impedance, so that they may create problems of instability for the antenna system housed inside them.

The invention intends solving this important technical problem with the following proposed solution. A first problem to be solved is that of choosing a type of antenna suitable for the abovementioned working frequencies, requiring the formation of a single opening of limited size in the cover of the metal box-shaped body of the implantable device, and for this purpose an antenna of the cylindrical helix type has been chosen. The second problem to be solved is that of preventing contact of the antenna or the antenna section outside the cover of the metal container with the environment inside the resin or silicone cap which is situated above the said cover. This problem has been solved by housing the external part of the antenna inside a hood which is made of a material which is gas-proof and moisture-proof but permeable to the working radiofrequency and can be fixed in a fluid-tight manner to the said titanium cover. According to a preferred embodiment of the invention, the said hood is made of ceramic material, namely alumina or compounds thereof, and the said hood is arranged on the base in any way such that it may be welded in a fluid-tight manner to the cover of the titanium container of the implantable device.

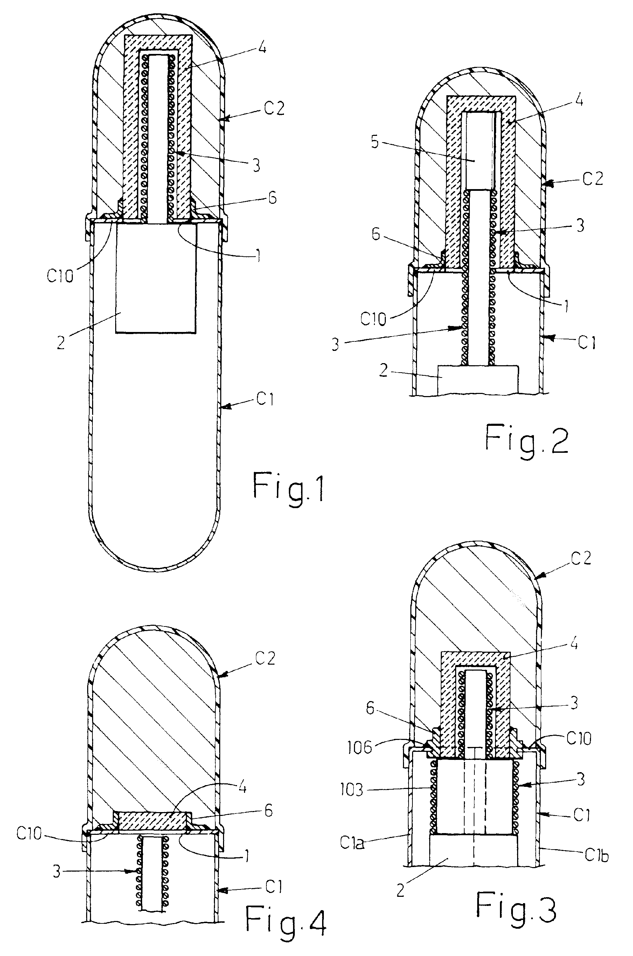

Further characteristic features of the invention and the advantages arising therefrom will appear more clearly from the following description of some preferred embodiments thereof, illustrated purely by way of a non-limiting example and with parts sectioned in Figures 1 to 4 of the single accompanying illustrative plate.

In Figure 1, C1 denotes the titanium box-shaped body of the implantable device and C2 denotes the resin or silicone rubber cap. According to the invention, the cover C10 of the container C1 has, formed therein, at least one window 1 of suitable size and shape, for example round, for allowing communication, with the exterior, of the antenna system which comprises an interface 2 inside the container C1 and at least any one suitable cylindrical-helix antenna 3 which, as in the version according to Figure 1, may be positioned completely outside the cover and therefore completely inside the cap C2 or, as shown in the solutions according to Figures 2 and 3, may be arranged partly inside the cap C2 and partly inside the container C1, with the part 103 inside this container which may have a diameter greater than the outer part, or finally, as in the solution shown in Figure 4, may be arranged completely inside the container C1. According to the invention, the hole 1 for receiving the antenna or allowing through the electric field produced by the antenna or directed towards it is closed by an insert 4 which is made of any material which is gas-proof and moisture-proof, but permeable to the radiofrequencies in question (402-405 MHz) and which may be fixed in a fluid-tight manner to the cover C10 using any technique suitable for the purpose. According to a preferred embodiment, the insert 4 is made of ceramic material, i.e. a material essentially based on alumina or derivatives thereof, or equivalent materials. The insert 4 may take the form of a hood of different heights, as shown in Figures 1 to 3, so as to cover the section of varying height of the antenna which projects from the container C1 or may have a substantially disk-shaped form as in the solution according to Figure 4 where the antenna is completely housed inside the container C1, since brief tests carried out have not excluded the functionality of this solution. The hood 4 shown in Figure 2 has the same height as that shown in Figure 1, although it houses inside it a section of antenna having a height less than that of Figure 1. In this case, the space of the hood 4 not occupied by the antenna is occupied by a resinous insert 5.

The constructional form of the antenna is not considered here since it will be easily determined experimentally by persons skilled in the art. Differently to that shown, the antenna may be made using a process similar to that for forming electronic printed circuits, with the deposition of metal on the inner side surface of the hood 4 or on the outer side surface of inserts made of suitable material and housed inside the said hood 4.

The insert 4 of ceramic material may be fixed to the cover C10 using any technique suitable for allowing a fluid-tight connection through which fluids cannot pass. Purely by way of a non-limiting example, good results have been obtained by fixing to the base of the insert 4 with a round cross-section, for example onto its outer side surface, for example by means of braze-welding, the collar of a metal ring 6 which is in turn welded using any technique to the cover C10, for example using the same technique with which the cover C10 is fixed to the container C1. In the solution shown in Figure 3, the ring 6 has externally an annular groove 106 inside which the opposite sides of the cover C10 are inserted when these are formed as one piece with the two shells C1 a and C1 b which form the titanium container of the implantable device.

## Claims

1. Antenna system for electro-medical devices which can be implanted in the human body, such as pacemakers, defibrillators or the like, so as to allow dialogue between the transceiver system inside these said devices and transceiver devices situated outside and also relatively distant from the patient's body, for example for the remote reception/transmission of data to and from monitoring stations or to and from control and/or programming stations, the said implantable devices having a box-shaped body (C1) which is made for example of titanium and contains the various electronic components and which is closed by a cover (C10) provided with openings from which the terminals for the stimulation and-/or control electrode or electrodes project, this cover having situated above it and being sealed in a fluid-tight manner by a cap (C2) which is made of resin or silicone or derivatives thereof and through which said terminals pass in a fluid-tight manner, **characterized in that** it comprises at least one antenna of the cylindrical helix type (3) able to operate at frequencies of between 402 and 405 MHz and also **characterized in that** on the cover (C10) of the said container (C1) there is formed at least one window (1) of suitable size and shape for allowing communication, with the exterior, of the said antenna system which comprises an interface (2) inside the container (C1) and the said cylindrical-helix antenna (3) which may be housed completely inside the resinous cap (C2) or partly inside the resinous cap and partly inside the metallic container (C1) or completely inside this container, the said hole (1) for receiving the antenna or allowing through the electric field produced by the said antenna or directed towards it being closed by an insert (4) which is made of any gas-proof and moisture-proof material permeable to the radiofrequencies in question and can be fixed in a fluid-tight manner on the said cover (C10).

2. Antenna system according to Claim 1), **characterized in that** the said insert (4) is made of ceramic material, i.e. a material essentially based on alumina or derivatives thereof or equivalent materials.

3. Antenna system according to Claim 1), **characterized in that** the said insert (4) has a monolithic constructional design and may have the form of a hood or the simple form of a stopper.

4. Antenna system according to claim 1), **characterized in that** the said insert (4) may be fixed onto the cover (C10) of the titanium container (C1) using a welding process.

5. Antenna system according to Claim 4), **characterized in that** the ceramic insert (4) has a round cross-section and has fixed thereto the collar of a metal ring (6) which is in turn fixed by means of welding to the cover (C10) of the metal container (C1).

6. Antenna system according to claim 1), **characterized in that** the antenna may be formed entirely or partly by means of electrolytic deposition of metal onto the inner surface of the ceramic insert (4) or onto the outer surface of a suitable support made of ceramic material or other material placed inside the said insert.
